# EUROPEAN PATENT APPLICATION

(11) **EP 3 800 178 A1**
(43) Date of publication of application: **07.04.2021**
(21) Application number: 19382841.5
(22) Date of filing: 01.10.2019
(51) Int. Cl.: C07C 211/42, C07B 43/06

(54) **PREPARATION OF ENAMIDE INTERMEDIATE FOR THE SYNTHESIS OF DASOTRALINE**

(71) Applicant: Moehs Ibérica, S.L., 08191 Rubi, Barcelona (ES)
(72) Inventor: BALLETTE, Roberto, E-08191 Rubi, Barcelona (ES); DOBARRO RODRÍGUEZ, Alicia, E-08191 Rubi, Barcelona (ES)
(74) Representative: ABG Intellectual Property Law, S.L.

(57) **Abstract**

The present invention relates to a process for the preparation of *N*-((*S*)-4-(3,4-dichlorophenyl)-3,4-dihydronaphthalen-1-yl)acetamide, which is a suitable intermediate for the synthesis of dasotraline or a pharmaceutically acceptable salt thereof, as well as to a process for the preparation of dasotraline or a pharmaceutically acceptable salt thereof using said *N*-((*S*)-4-(3,4-dichlorophenyl)-3,4-dihydronaphthalen-1-yl)acetamide intermediate.

## Description

### FIELD OF THE INVENTION

The present invention relates to a process for the preparation of *N*-((*S*)-4-(3,4-dichlorophenyl)-3,4-dihydronaphthalen-1-yl)acetamide, which is an intermediate in the synthesis of dasotraline or a pharmaceutically acceptable salt thereof, as well as to a process for the preparation of dasotraline or a pharmaceutically acceptable salt thereof using said intermediate.

### BACKGROUND OF THE INVENTION

Dasotraline is a dual dopamine and norepinephrine reuptake inhibitor developed by Sunovion for the treatment of attention deficit hyperactivity disorder and moderate to severe binge eating disorder.

Dasotraline is also known as (1*R*,4*S*)-*trans*-norsertraline or (1*R*,4*S*)-4-(3,4-dichlorophenyl)-1,2,3,4-tetrahydronaphthalen-1-amine and has the chemical structure (III) depicted below.

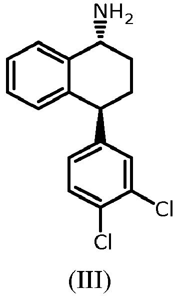

Dasotraline has been disclosed in patent document WO 2004/024669 A1. This document describes that dasotraline (III) is obtained by reaction of (*S*)-4-(3,4-dichlorophenyl)-3,4-dihydro-1-naphthalenone (II) with an excess of formic acid and formamide to give a mixture of (1*S*,4*S*)- and (1*R*,4*S*)-*N*-[4-(3,4-dichlorophenyl)-1,2,3,4-tetrahydronaphthanlen-1-yl]formamide (V), followed by a purification step by flash column chromatography to separate the (1*R*,4*S*)-distereoisomer (Va) from the (1*S*,4*S*)-diastereoisomer (Vb), and hydrolysis of the (1*R*,4*S*)-formamide diastereoisomer (Va) with aqueous acid to give dasotraline hydrochloride (III)·HCl, as shown below in Scheme 1. This process is not suitable for industrial production since it comprises a chromatographic purification step.

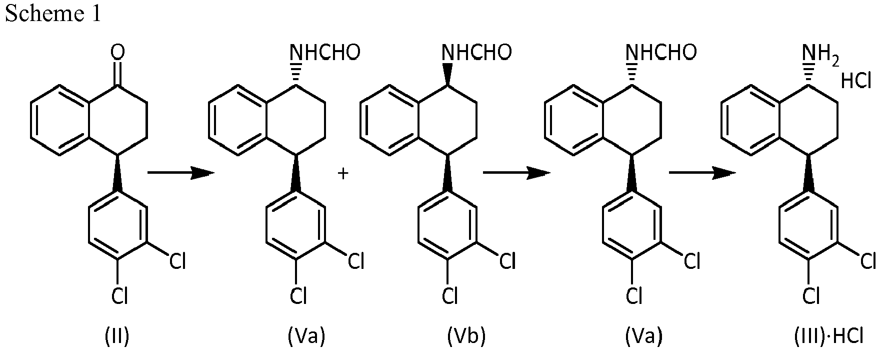

Patent document WO 2007/115185 A2 discloses dasotraline and a process for its preparation which is suitable for large-scale preparation. This patent document describes a process for the conversion of (*S*)-4-(3,4-dichlorophenyl)-3,4-dihydro-1-naphthalenone (II) to the corresponding oxime (VI) by reaction with hydroxylamine hydrochloride and sodium acetate, followed by contacting said oxime (VI) with a phosphine (triethylphosphine or tri-n-butylphoshine are used in example 1.2) and an acyl donor (acetic anhydride is used in example 1.2) to convert oxime (VI) into enamide (I), as shown below in Scheme 2. Document WO 2007/115185 A2 further discloses the conversion of enamide (I) into dasotraline by stereoselective hydrogenation of the double bond of enamide (I) in the presence of a chiral catalyst ((*R,R*)-MeBPE(COD)RhBF₄ in examples 2 and 6, which is a complex of rhodium with the chiral phosphine ligand (2*R*,5*R*)-1,2-bis(2,5-dimethylphospholano)ethane (MeBPE); (*R,S,R,S*)-MePenn Phos(COD)RhBF₄ in example 5, which is a complex of rhodium with the chiral phosphine ligand (2*R*,5*S*,2*R*,5*S*)-P,P-1,2-phenylenebis{(2,5-*endo*-dimethyl)-7-phosphabicyclo [2.2.2]heptane} (R,S,R,S-MePennPhos); and (*R,R*)Norphos(COD)RhBF₄ in example 7, which is a complex of rhodium with the chiral phosphine ligand (2*R,*3*R*)-5,5-bis(diphenylphosphino)bicycle[2.2.1]hept-2-ene (Norhpos)) to give acetamide (IV), followed by amide deprotection to yield dasotraline (III), as shown in Scheme 2 below.

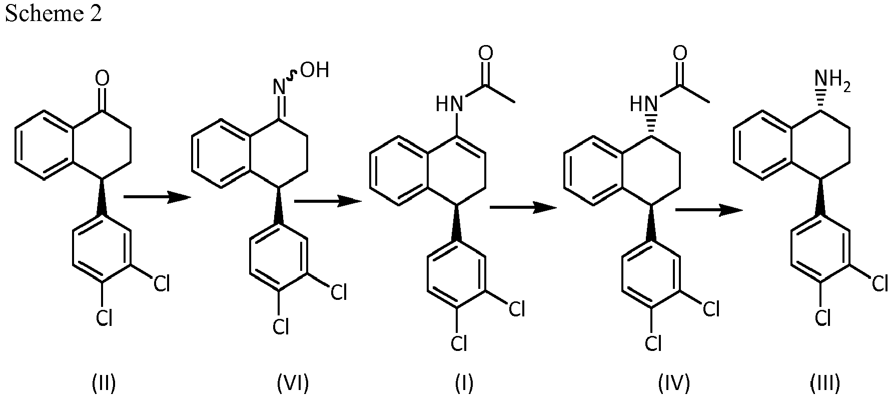

Patent document WO 2018/144385 A1 relates to an improvement (acceleration of the reaction rate) of the process of WO 2007/115185 A2 described above for the conversion of an oxime, such as compound (VI), to the corresponding enamide, such as compound (I), which can be used for the manufacture of dasotraline. The process disclosed in WO 2018/144385 A1 comprises contacting an oxime, such as compound (VI), with an acyl donor and a phosphine in the presence of an iron reagent that provides from 10 to 2000 ppm iron, such as FeCl₂ and Fe(OAc)₂, to give the corresponding enamide, such as compound (I). This enamide is then subjected to catalytic stereoselective hydrogenation to give the corresponding amide, such as compound (IV), which upon deprotection of the acetamide group provides the target dasotraline of formula (III). The reaction scheme of this process corresponds to the same as depicted above in Scheme 2. The variation with respect to the process disclosed in WO 2007/115185 A2 lies only in the addition of catalytic amounts of iron in the conversion from oxime (VI) to enamide (I) which is reported in WO 2018/144385 A1 as accelerating the reaction rate.

Despite the availability of processes suitable for industrial scale production of dasotraline, such as those disclosed in WO 2007/115185 A2 and WO 2018/144385 A1, there remains a need for a new suitable processes, in particular which provides dasotraline in high yield and diastereomeric purity.

### SUMMARY OF THE INVENTION

The inventors have surprisingly found a process for the production of dasotraline in high yield and diastereomeric purity, which provides the enamide intermediate of formula (I) using mild reaction conditions, a single reaction vessel, and with simple isolation of said enamide intermediate (I) from the reaction mixture. Said enamide intermediate (I) can then be used for the synthesis of dasotraline or a pharmaceutically acceptable salt thereof.

Thus, in a first aspect, the present invention relates to a process for the preparation of *N-*((*S*)-4-(3,4-dichlorophenyl)-3,4-dihydronaphthalen-1-yl)acetamide of formula (I) 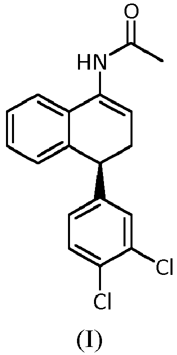 comprising:
a) reacting (*S*)-4-(3,4-dichlorophenyl)-3,4-dihydro-1-naphthalenone of formula (II) with an ammonia source in the presence of a titanium(IV) C₂-C₄ alkoxide 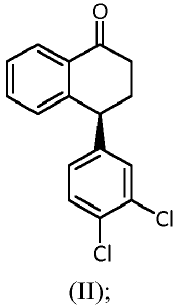
b) reacting the product obtained step a) with an acetyl donor in the presence of a base selected from the group consisting of C₁-C₄ alkylamine and C₁-C₄-dialkylamine to give the *N*-((*S*)-4-(3,4-dichlorophenyl)-3,4-dihydronaphthalen-1-yl)acetamide of formula (I). and
c) optionally isolating the *N*-((*S*)-4-(3,4-dichlorophenyl)-3,4-dihydronaphthalen-1-yl)acetamide of formula (I) obtained in step b).

In a second aspect, the present invention relates to a process for the preparation of dasotraline of formula (III) 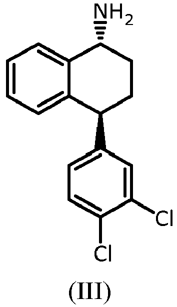 or a pharmaceutically acceptable salt thereof, said process comprising:
i) carrying out the process defined in the first aspect, thereby obtaining *N*-((*S*)-4-(3,4-dichlorophenyl)-3,4-dihydronaphthalen-1-yl)acetamide of formula (I) 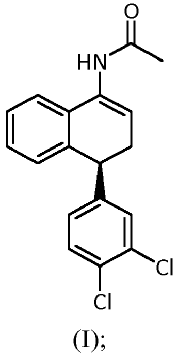
ii) hydrogenating the *N*-((*S*)-4-(3,4-dichlorophenyl)-3,4-dihydronaphthalen-1-yl)acetamide of formula (I) obtained in step i) with hydrogen in the presence of a chiral hydrogenation catalyst, thereby obtaining *N*-((1*R*,4*S*)-4-(3,4-dichlorophenyl)-3,4-dihydronaphthalen-1-yl)acetamide of formula (IV) 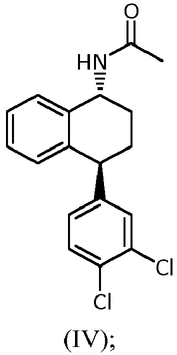 and
iii) deacetylating *N*-((1*R*,4*S*)-4-(3,4-dichlorophenyl)-3,4-dihydronaphthalen-1-yl)acetamide of formula (IV) in the presence of a deacetylating agent, thereby obtaining dasotraline or a pharmaceutically acceptable salt thereof.

### DESCRIPTION OF THE INVENTION

### Preparation of N-((S)-4-(3,4-dichlorophenyl)-3,4-dihydronaphthalen-1-yl)acetamide (I)

In the first aspect, the present invention relates to a process for the preparation of *N-*((*S*)-4-(3,4-dichlorophenyl)-3,4-dihydronaphthalen-1-yl)acetamide of formula (I) 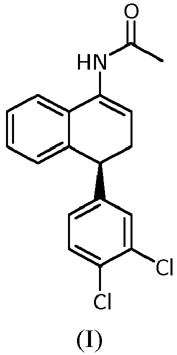 comprising:
a) reacting (*S*)-4-(3,4-dichlorophenyl)-3,4-dihydro-1-naphthalenone of formula (II) with an ammonia source in the presence of a titanium(IV) C₂-C₄ alkoxide 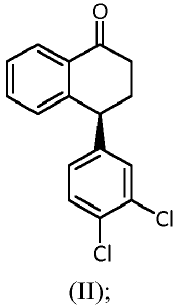
b) reacting the product obtained step a) with an acetyl donor in the presence of a base selected from the group consisting of C₁-C₄ alkylamine and C₁-C₄-dialkylamine to give the *N*-((*S*)-4-(3,4-dichlorophenyl)-3,4-dihydronaphthalen-1-yl)acetamide of formula (I). and
c) optionally isolating the *N*-((*S*)-4-(3,4-dichlorophenyl)-3,4-dihydronaphthalen-1-yl)acetamide of formula (I) obtained in step b).

Step a) of the process is reacting (*S*)-4-(3,4-dichlorophenyl)-3,4-dihydro-1-naphthalenone of formula (II) with an ammonia source in the presence of a titanium(IV) C₂-C₄ alkoxide.

The term "ammonia source" refers to any compound or mixture of compounds capable of providing NH₃. Examples of ammonia sources are ammonia (as such, NH₃) and mixtures of an ammonium salt and a base. When mixtures of an ammonium salt and a base are used, the base is preferably provided at least in an equimolar amount with respect to the ammonium salt.

The term "ammonium salt" refers to a compound formed by an NH₄⁺ cation and a suitable counter ion such as Br⁻, Cl⁻, CO₃²⁻ and the like. Thus, examples of ammonium salts are NH₄Br, NH₄Cl, (NH₄)₂CO₃ and the like, preferably NH₄Br.

The term "base" refers to a compound having a pkₐ (relative to water) of the corresponding conjugate acid of from 5 to less than 14. Examples of suitable bases are organic amines, such as alkylamines, including secondary and tertiary amines, wherein each alkyl moiety is independently selected from a C₁-C₆ alkyl group, preferably a C₁-C₄ alkyl group (such as triethylamine, diethylamine, diisopropylethylamine, *tert-*butylamine and the like), inorganic bases such as alkali metal or alkaline earth metal hydrogen carbonates (such as NaHCO₃), alkali metal or alkaline earth metal carbonates (such as Na₂CO₃, K₂CO₃), alkali metal hydroxides (such as sodium hydroxide and potassium hydroxide). Preferably, the base is an organic amine selected from the group consisting of triethylamine, diethylamine, diisoproylethylamine and *tert*-butylamine more preferably triethylamine.

Preferably, the ammonia source is selected from the group consisting of ammonia and NH₄Br/trimethylamine. More preferably, the ammonia source is ammonia.

The term "C₂-C₄ alkoxide" refers to an alkyl group having from 2 to 4 carbon atoms, linear or branched, bound to a negatively charged oxygen atom (-O⁻), such as ethoxide, *n*-propoxide, isopropoxide, *n*-butoxide, isobutoxide, *sec*-butoxide and *tert*-butoxide, preferably isopropoxide or ethoxide.

The term "alkyl" refers to a refers to a straight or branched hydrocarbon chain radical consisting of carbon and hydrogen atoms, containing no unsaturation, having the number of carbon atoms preceding the term, and which is attached to the rest of the molecule by a single bond.

Thus, the term "titanium(IV) C₂-C₄ alkoxide" refers to titanium(IV) ethoxide, titanium(IV) n-propoxide, titanium(IV) isopropoxide, titanium(IV) *n*-butoxide, titanium(IV) isobutoxide, titanium(IV) *sec*-butoxide and titanium(IV) *tert*-butoxide, preferably titanium(IV) isopropoxide or titanium(IV) ethoxide, more preferably titanium(IV) isopropoxide.

Preferably, the titanium(IV) C₂-C₄ alkoxide is titanium (IV) isopropoxide and the ammonia source is ammonia (as such, NH₃).

Preferably, the molar ratio of titanium(IV) C₂-C₄ alkoxide, in particular titanium(IV) isopropoxide, to (*S*)-4-(3,4-dichlorophenyl)-3,4-dihydro-1-naphthalenone of formula (II) used in step a) is from 1.5:1 to 2.5:1, more preferably from 1.8:1 to 2.2:1, even more preferably from 1.9:1 to 2.1:1.

The definition of a molar ratio of two compounds A to B as being, for example, 1.5:1 means that the amount of moles of compound A is 1.5 times the amount of moles of compound B.

Preferably, the molar ratio of ammonia source, in particular ammonia, to (*S*)-4-(3,4-dichlorophenyl)-3,4-dihydro-1-naphthalenone of formula (II) used in step a) is from 1.1:1 to 2.0:1, more preferably from 1.2:1 to 1.8:1, even more preferably 1.4:1 to 1.6:1.

When the ammonia source is an ammonium salt and a base, the molar ratio defined above, is calculated with respect to the moles of ammonia provided, which generally corresponds to the moles of ammonium salt.

Preferably, step a) is carried out in a solvent selected from C₁-C₄ alkanol,

The term "C₁-C₄ alkanol" refers to an alkyl group having from 1 to 4 carbon atoms, linear or branched, bound to one hydroxyl group (-OH). The term "alkyl" is as previously defined.

Examples of C₁-C₄ alkanols are methanol, ethanol, *n*-propanol, isopropanol, *n*-butanol, isobutanol, sec-butanol, *tert*-butanol or mixtures thereof, more preferably methanol.

In a particular embodiment, step a) is carried out by mixing the (S)-4-(3,4-dichlorophenyl)-3,4-dihydro-1-naphthalenone of formula (II), ammonia source (in particular ammonia) and titanium(IV) C₂-C₄ alkoxide (in particular titanium(IV) isopropoxide), preferably in the presence of a solvent selected from C₁-C₄ alkanol, such as methanol, ethanol, *n*-propanol, isopropanol, *n*-butanol, isobutanol, sec-butanol, *tert-*butanol or mixtures thereof, more preferably methanol, at a temperature of from 15 °C to 30 °C, preferably from 20 °C to 25 °C. Preferably, this mixing is carried out for 24 h to 72 h, more preferably from 36 h to 60 h, even more preferably from 45 h to 50 h.

In a particular embodiment, step a) is carried out by first mixing the (S)-4-(3,4-dichlorophenyl)-3,4-dihydro-1-naphthalenone of formula (II) and ammonia source (in particular ammonia), and then adding titanium(IV) C₂-C₄ alkoxide (in particular titanium(IV) isopropoxide), preferably in the presence of a solvent selected from C₁-C₄ alkanol, such as methanol, ethanol, *n*-propanol, isopropanol, *n*-butanol, isobutanol, sec-butanol, *tert*-butanol or mixtures thereof, more preferably methanol. The addition of titanium(IV) C₂-C₄ alkoxide (in particular titanium(IV) isopropoxide) is preferably carried out at a temperature of from -5 °C to 5 °C, and once the addition is completed, the mixture is kept at a temperature of from 15 °C to 30 °C, preferably from 20 °C to 25 °C. Preferably, this mixing a temperature of from 15 °C to 30 °C, preferably from 20 °C to 25 °C, is carried out for 24 h to 72 h, more preferably from 36 h to 60 h, even more preferably from 45 h to 50 h.

Step b) of the process is reacting the product obtained step a) with an acetyl donor in the presence of a base selected from the group consisting of C₁-C₄ alkylamine and C₁-C₄-dialkylamine to give the *N*-((*S*)-4-(3,4-dichlorophenyl)-3,4-dihydronaphthalen-1-yl)acetamide of formula (I).

The term "acetyl donor" refers to any chemical compound capable of transferring an acetyl group (CH₃C(=O)-, which is abbreviated as Ac) from itself to another molecular entity. Examples of acetyl donors are acetic anhydride and acetyl chloride, preferably the acetyl donor is acetic anhydride.

The term "base" refers to a compound having a pkₐ (relative to water) of the corresponding conjugate acid of from 5 to less than 14. The base is selected from C₁-C₄ alkylamines and C₁-C₄-dialkylamines.

The term "C₁-C₄ alkylamine" refers to a compound of formula R-NH₂, wherein R is a C₁-C₄ alkyl group, i.e. an alkyl as previously defined having from 1 to 4 carbon atoms.

The term "C₁-C₄ dialkylamine" refers to a compound of formula R-NH-R', wherein each R and R' is independently selected from a C₁-C₄ alkyl group, i.e. an alkyl as previously defined having from 1 to 4 carbon atoms.

Examples of suitable C₁-C₄ alkylamines and C₁-C₄-dialkylamines are triethylamine, diethylamine, diisopropylethylamine, *tert*-butylamine and the like, preferably triethylamine.

In a preferred embodiment, the acetyl donor in step b) is acetic anhydride and the base is triethylamine.

Preferably, the molar ratio of acetyl donor used in step b) to (*S*)-4-(3,4-dichlorophenyl)-3,4-dihydro-1-naphthalenone of formula (II) used in step a) is from 1.5:1 to 2.5:1, more preferably from 1.8:1 to 2.2:1, even more preferably from 1.9:1 to 2.1:1.

Preferably, the molar ratio of base used in step b) to (*S*)-4-(3,4-dichlorophenyl)-3,4-dihydro-1-naphthalenone of formula (II) used in step a) is from 2:1 to 5:1, more preferably from 3:1 to 5:1, even more preferably from 3.5:1 to 4.5:1.

Preferably, the molar ratio of acetyl donor used in step b) to (*S*)-4-(3,4-dichlorophenyl)-3,4-dihydro-1-naphthalenone of formula (II) used in step a) is from 1.5:1 to 2.5:1 and the molar ratio of base used in step b) to (*S*)-4-(3,4-dichlorophenyl)-3,4-dihydro-1-naphthalenone of formula (II) used in step a) is from 2:1 to 5:1. More preferably the molar ratio of acetyl donor used in step b) to (*S*)-4-(3,4-dichlorophenyl)-3,4-dihydro-1-naphthalenone of formula (II) used in step a) is from 1.8:1 to 2.2:1 and the molar ratio of base used in step b) to (*S*)-4-(3,4-dichlorophenyl)-3,4-dihydro-1-naphthalenone of formula (II) used in step a) is from 3:1 to 5:1. Even more preferably the molar ratio of acetyl donor used in step b) to (*S*)-4-(3,4-dichlorophenyl)-3,4-dihydro-1-naphthalenone of formula (II) used in step a) is from 1.9:1 to 2.1:1 and the molar ratio of base used in step b) to (*S*)-4-(3,4-dichlorophenyl)-3,4-dihydro-1-naphthalenone of formula (II) used in step a) is from 3.5:1 to 4.5:1.

Preferably, step b) is carried out in a solvent selected from C₁-C₄ alkanol, such as methanol, ethanol, *n*-propanol, isopropanol, *n*-butanol, isobutanol, sec-butanol, *tert-*butanol or mixtures thereof, more preferably methanol.

In particular, steps a) and b) are carried out in the same solvent, which is selected from a C₁-C₄ alkanol, such as methanol, ethanol, *n*-propanol, isopropanol, *n*-butanol, isobutanol, sec-butanol, *tert*-butanol or mixtures thereof, more preferably methanol.

In a particular embodiment, step b) is carried out by mixing the product obtained step a) with the acetyl donor and the base, preferably in the presence of a solvent selected from C₁-C₄ alkanol, such as methanol, ethanol, *n*-propanol, isopropanol, *n*-butanol, isobutanol, sec-butanol, *tert*-butanol or mixtures thereof, more preferably methanol, at a temperature of from 15 °C to 30 °C, preferably from 20 °C to 25 °C. Preferably, this mixing is carried out for 12 h to 48 h, more preferably from 12 h to 36 h, even more preferably from 20 h to 30 h.

In a particular embodiment, step b) is carried out by first mixing the product obtained step a) with the base and then adding the acetyl donor, preferably in the presence of a solvent selected from C₁-C₄ alkanol, such as methanol, ethanol, *n*-propanol, isopropanol, *n*-butanol, isobutanol, sec-butanol, *tert*-butanol or mixtures thereof, more preferably methanol. The addition of the acetyl donor is preferably carried out at a temperature of from -5 °C to 10 °C, preferably from 0 °C to 5 °C, and once the addition is completed, the mixture is kept at a temperature of from 15 °C to 30 °C, preferably from 20 °C to 25 °C. Preferably, this mixing a temperature of from 15 °C to 30 °C, preferably from 20 °C to 25 °C, is carried out for 12 h to 48 h, more preferably from 12 h to 36 h, even more preferably from 20 h to 30 h.

Preferably, the process for the preparation of *N*-((*S*)-4-(3,4-dichlorophenyl)-3,4-dihydronaphthalen-1-yl)acetamide of formula (I) further comprises step c) of isolating the *N*-((*S*)-4-(3,4-dichlorophenyl)-3,4-dihydronaphthalen-1-yl)acetamide of formula (I) obtained in step b).

In particular, the isolation of step c) comprises:
c1) treating the reaction mixture obtained in step b) with *N,N,N',N'*-tetrakis(2-hydroxy-C₂-C₃ alkyl)ethylenediamine;
c2) adding a basic aqueous solvent and a non-water-miscible organic solvent to the mixture obtained in c1);
c3) separating the organic phase;
c4) removing the solvent from the organic phase, thereby obtaining isolated *N*-((*S*)-4-(3,4-dichlorophenyl)-3,4-dihydronaphthalen-1-yl)acetamide of formula (I); and
c5) optionally recrystallizing the isolated *N*-((*S*)-4-(3,4-dichlorophenyl)-3,4-dihydronaphthalen-1-yl)acetamide of formula (I).

In particular, the isolation of step c) comprises:
c1) treating the reaction mixture obtained in step b) with *N,N,N',N'*-tetrakis(2-hydroxy-C₂-C₃ alkyl)ethylenediamine;
c2) adding a basic aqueous solvent and a non-water-miscible organic solvent to the mixture obtained in c1);
c3) separating the organic phase from the mixture obtained in step c2);
c4) removing the solvent from the organic phase obtained in c3), thereby obtaining isolated *N*-((*S*)-4-(3,4-dichlorophenyl)-3,4-dihydronaphthalen-1-yl)acetamide of formula (I); and
c5) optionally recrystallizing the isolated *N*-((*S*)-4-(3,4-dichlorophenyl)-3,4-dihydronaphthalen-1-yl)acetamide of formula (I) obtained in step c4).

These isolation steps are advantageous for the removal of Ti-related by-products since they avoid filtration processes which are usually slow. These isolation steps allow the separation of Ti-related by-products by extraction.

Preferably, the *N,N,N',N'*-tetrakis(2-hydroxy-C₂-C₃ alkyl)ethylenediamine is selected from the group consisting of *N,N,N',N'*-tetrakis(2-hydroxyethyl)ethylenediamine and *N,N,N',N'*-tetrakis(2-hydroxypropyl)ethylenediamine and mixtures thereof, more preferably *N,N,N',N'*-tetrakis(2-hydroxyethyl)ethylenediamine.

Preferably, the molar ratio of *N,N,N',N'*-tetrakis(2-hydroxy-C₂-C₃ alkyl)ethylenediamine used in step c1) to titanium(IV) C₂-C₄ alkoxide (in particular titanium(IV) isopropoxide) used in step a) is from 1.0:1 to 2.0:1, more preferably from 1.0:1 to 1.5:1, even more preferably from 1.0:1 to 1.2:1, still more preferably from 1.0:1 to 1.1:1.

Step c1) is carried out by adding *N,N,N',N'*-tetrakis(2-hydroxy-C₂-C₃ alkyl)ethylenediamine to the reaction mixture obtained in step b). The addition is preferably carried out at a temperature of from 15 °C to 30 °C, preferably from 20 °C to 25 °C. Preferably, the resulting mixture is kept at a temperature of from 15 °C to 30 °C, preferably from 20 °C to 25 °C, for 0.5 h to 2 h, more preferably from 0.5 h to 1.5 h, even more preferably from 1 h to 1.5 h.

Step c2) involves adding a basic aqueous solvent and a non-water-miscible organic solvent. These solvents are added to the mixture obtained in step c1) to carry out an extraction process to separate the target *N-*((*S*)-4-(3,4-dichlorophenyl)-3,4-dihydronaphthalen-1-yl)acetamide of formula (I).

The term "aqueous solvent" refers to a solvent having at least 60% by volume of water with respect to the total volume of the aqueous solvent, at least 65%, at least 70%, or at least 75%. The term "basic aqueous solvent" refers to an aqueous solvent as previously defined further comprising a base, preferably ammonia. The term "base" in said basic aqueous solvent refers to a compound having a pkₐ (relative to water) of the corresponding conjugate acid of from 5 to less than 14. Examples of suitable bases are ammonia, organic amines, such as alkylamines, including secondary and tertiary amines, wherein each alkyl moiety is independently selected from a C₁-C₆ alkyl group, preferably a C₁-C₃ alkyl group (such as triethylamine, diethylamine, diisopropylethylamine and the like), inorganic bases such as alkali metal or alkaline earth metal hydrogen carbonates (such as NaHCO₃), alkali metal or alkaline earth metal carbonates (such as Na₂CO₃, K₂CO₃), alkali metal hydroxides (such as sodium hydroxide and potassium hydroxide). Preferably, the base is ammonia.

Preferably, the basic aqueous solvent of step c2) is a mixture of water and ammonia, more preferably in a volume ratio of from 5:1 to 2:1, even more preferably from 4:1 to 2:1, still more preferably from 3.5:1 to 2.5:1.

The term "non-water-miscible organic solvent" refers to an organic solvent which is not miscible with water in at least one proportion of said solvent and water, i.e. in at least one proportion of the solvent and water two phases are produced. These phases can be easily identified by visual inspection. In contrast, a "water miscible" solvent refer to a solvent that is completely miscible with water at any proportion of the solvent and water, i.e. no phase separation is produced. Miscibility is considered at 20 °C to 25 °C. Examples of non-water-miscible organic solvents are ethyl acetate, butyl acetate, methyl ethyl ketone, methyl-*tert*-butyl ether, diisopropylether dichloromethane, chloroform, cyclohexane, hexane, heptane, pentane, toluene, xylene and the like, preferably ethyl acetate.

Preferably, the non-water-miscible organic solvent of step c2) is ethyl acetate.

Preferably, the basic aqueous solvent of step c2) is a mixture of water and ammonia and the non-water-miscible organic solvent of step c2) is ethyl acetate.

In particular, the volume ratio of basic aqueous solvent to non-water-miscible organic solvent is from 2:1 to 0.8:1, more preferably from 1.5:1 to 1:1, even more preferably from 1.3:1 to 1.1:1.

After mixing, the phases are allowed to separate and then step c3) of separating the organic phase from the mixture obtained in step c2) is performed.

After step c3), step c4) is performed. Step c4) is the removal of the solvent from the organic phase obtained in the previous step, thereby obtaining isolated *N*-((*S*)-4-(3,4-dichlorophenyl)-3,4-dihydronaphthalen-1-yl)acetamide of formula (I).

Removal of the solvent means removing at least 80% by volume with respect to the volume or solvent present in the organic phase before removal of the solvent, removing at least 85%, preferably at least 90%, more preferably at least 95%. Removal of the solvent may be carried out by conventional means in the art, such as evaporating at reduced pressure.

Isolated *N*-((*S*)-4-(3,4-dichlorophenyl)-3,4-dihydronaphthalen-1-yl)acetamide of formula (I) refers to *N*-((*S*)-4-(3,4-dichlorophenyl)-3,4-dihydronaphthalen-1-yl)acetamide of formula (I) which has been substantially separated from reaction by-products, reagents, catalysts and/or solvents. However, the presence of minor amounts of these reaction by-product, reagents, catalysts and/or solvents is allowed. Isolated N-((*S*)-4-(3,4-dichlorophenyl)-3,4-dihydronaphthalen-1-yl)acetamide of formula (I) preferably refers to *N*-((*S*)-4-(3,4-dichlorophenyl)-3,4-dihydronaphthalen-1-yl)acetamide of formula (I) having a purity of at least 80%, more preferably of at least 85%, more preferably of at least 90%, even more preferably of at least 95%. The purity of *N*-((*S*)-4-(3,4-dichlorophenyl)-3,4-dihydronaphthalen-1-yl)acetamide of formula (I) may be determined by Ultra High Performance Liquid Chromatography (UHPLC) using the method described in the examples.

Then, step c5) of the process may be carried out. This is an optional step and which involves recrystallizing the isolated *N*-((*S*)-4-(3,4-dichlorophenyl)-3,4-dihydronaphthalen-1-yl)acetamide of formula (I) obtained in step c4). This recrystallization is preferably carried out in a mixture of heptane and toluene, preferably in a volume ratio of from 3:1 to 2:1 of heptane/toluene, more preferably from 2.5:1 to 2:1 of heptane/toluene, even more preferably from 2.4:1 to 2.3:1.

When preforming the process of the invention, *N*-((*S*)-4-(3,4-dichlorophenyl)-3,4-dihydronaphthalen-1-yl)acetamide of formula (I) obtained in high purity.

### Preparation of dasotraline of formula (III) or a pharmaceutically acceptable salt thereof

In a second aspect, the present invention relates to a process for the preparation of dasotraline of formula (III) 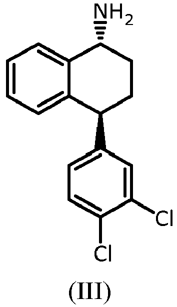 or a pharmaceutically acceptable salt thereof, said process comprising:
i) carrying out the process defined in the first aspect, thereby obtaining *N*-((*S*)-4-(3,4-dichlorophenyl)-3,4-dihydronaphthalen-1-yl)acetamide of formula (I) 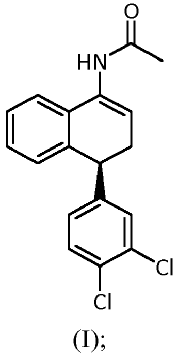
ii) hydrogenating the *N*-((*S*)-4-(3,4-dichlorophenyl)-3,4-dihydronaphthalen-1-yl)acetamide of formula (I) obtained in step i) with hydrogen in the presence of a chiral hydrogenation catalyst, thereby obtaining *N*-((1*R*,4*S*)-4-(3,4-dichlorophenyl)-3,4-dihydronaphthalen-1-yl)acetamide of formula (IV) 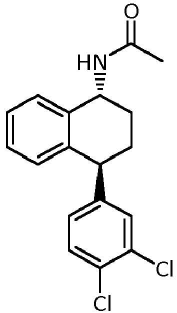 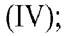 and
iii) deacetylating *N*-((1*R*,4*S*)-4-(3,4-dichlorophenyl)-3,4-dihydronaphthalen-1-yl)acetamide of formula (IV) in the presence of a deacetylating agent, thereby obtaining dasotraline or a pharmaceutically acceptable salt thereof.

The first step of this process, step i), is carrying out the process defined in the first aspect, as previously explained, thereby obtaining *N*-((*S*)-4-(3,4-dichlorophenyl)-3,4-dihydronaphthalen-1-yl)acetamide of formula (I).

The second step of this process, step ii), is hydrogenating the *N*-((*S*)-4-(3,4-dichlorophenyl)-3,4-dihydronaphthalen-1-yl)acetamide of formula (I) obtained in step i) with hydrogen in the presence of a chiral hydrogenation catalyst, thereby obtaining *N-*((1*R*,4*S*)-4-(3,4-dichlorophenyl)-3,4-dihydronaphthalen-1-yl)acetamide of formula (IV)

The hydrogenation reduces the double bond present in enamide (I). Thus, the resulting compound may have to different configuration of the newly generated chiral carbon atom bearing the acetamide substituent. Thus, to obtain the desired chiral configuration at said carbon atom, the hydrogenation needs to be performed in a stereoselective manner.

Thus, the hydrogenation of step ii) is carried out in the presence of a chiral hydrogenation catalyst. Suitable chiral hydrogenation catalysts are chiral complexes of a transition metal, such as rhodium, with a chiral phosphine ligand, including monodentate and bidentate ligands, such as 5,6-bis(diphenylphosphino)bicyclo[2.2.1]hept-2-ene (NorPhos), 1,2-bis(2,5-dimethylphospholano)ethane (MeBPE), P,P-1,2-phenylenebis {(2,5-*endo*-dimethyl)-7-phosphabicyclo[2.2.1]heptane} (MePennPhos), and 3,4-bis(diphenylphosphino)-*N-*benzylpyrrolidine (catASium® D), as described in WO 2007/115185 A2 and WO 2018/144385 A1. The structure of said phosphine ligands is depicted below.

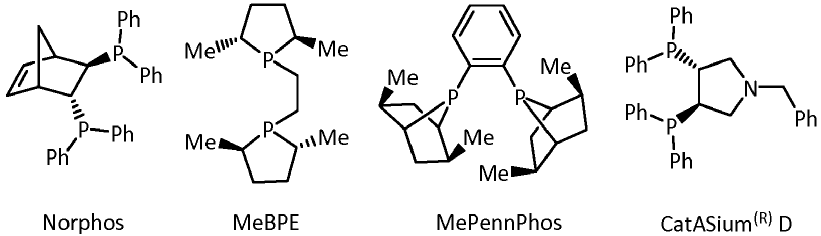

Preferably, the transition metal is rhodium and the chiral phosphine ligand is NorPhos.

Examples of chiral hydrogenation catalysts are (*R,R*)-Norphos(COD)RhBF₄ (i.e., ((2*R*,3*R*)-(+)-2,3-bis(diphenylphosphino)bicyclo[2.2.1]hept-5-en-(1,5-cyclooctadien) rhodium(I) tetrafluoroborate, (*R,S,R,S*)-MePennPhos(COD)RhBF₄ (i.e. (2*R*,5*S*,2*R*,5*S*)-P,P-1 ,2-phenylenebis {(2,5-endo-dimethyl)-7-phosphabicyclo[2.2.1]heptane}-(1,5-cyclooctadien) rhodium(I) tetrafluoroborate), (*R,R*)-MeBPE(COD)RhBF₄ (i.e. (2*R,*5*R*)-1,2-bis(2,5-dimethylphospholano)ethane}-(1,5-cyclooctadien) rhodium(I) tetrafluoroborate) and (*R,R*)-catASium® D(COD)RhBF₄ (i.e. (3*R*,4*R*)-3,4-bis(diphenylphosphino)-*N*-benzyl pyrrolidine-(1,5-cyclooctadien) rhodium(I) tetrafluoroborate). By way of example, the structure of (*R,R*)-Norphos(COD)RhBF₄ is depicted below.

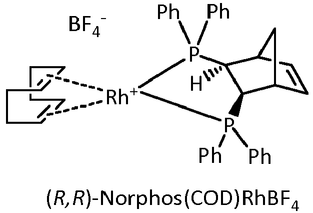

Preferably, the chiral hydrogenation catalysts is (*R,R*)-Norphos(COD)RhBF₄.

Preferably, the molar ratio of chiral hydrogenation catalyst to N-((S)-4-(3,4-dichlorophenyl)-3,4-dihydronaphthalen-1-yl)acetamide of formula (I) used in step ii) is from 0.00125:1 to 0.005:1, more preferably from 0.002:1 to 0.005:1, even more preferably from 0.002:1 to 0.003:1, even more preferably where the chiral hydrogenation catalyst is (*R,R*)-Norphos(COD)RhBF₄.

The hydrogenation may be carried out in any suitable solvent, which can be easily determined by the skilled person. Preferably, the solvent is selected from a C₁-C₄ alkanol. The term alkanol is as previously defined herein. Examples of C₁-C₄ alkanols are methanol, ethanol, *n*-propanol, isopropanol, *n*-butanol, isobutanol, *sec*-butanol, *tert-*butanol and mixtures thereof, preferably isopropanol and methanol, even more preferably isopropanol. When mixtures of different C₁-C₄ alkanols are used, these may comprise 2 or more different C₁-C₄ alkanols, such as 2, 3 or 4 C₁-C₄ alkanols, preferably 2 C₁-C₄ alkanols. When mixtures of different C₁-C₄ alkanols are used, these may be in any proportion. In particular, when a mixture of isopropanol and methanol is used, isopropanol is the main alkanol, thus it is present in more than 50% by volume with respect to the volume of the mixture of isopropanol and methanol, preferably in more than 60%, more preferably in more than 70%, more preferably in more than 80%, more preferably in more than 90%, more preferably in more than 95%, even more preferably in more than 97%.

The solvent may be present in any amount, in particular it is present in an amount that solubilizes the N-((S)-4-(3,4-dichlorophenyl)-3,4-dihydronaphthalen-1-yl)acetamide of formula (I).

Hydrogenation is usually carried out at a pressure of hydrogen of 1 to 8 bar, preferably 2 to 7 bar, more preferably 5 to 7 bar, even more permeably 5.5 to 6.5 bar.

Preferably, step ii) is carried out at a temperature of from 25 °C to 35 °C, preferably from 28 °C to 32 °C.

Preferably, the hydrogenation is carried out for 10 h to 24 h, more preferably from 12 h to 20 h, even more preferably from 12 h to 16 h.

This step, step ii), yields *N*-((1*R*,4*S*)-4-(3,4-dichlorophenyl)-3,4-dihydronaphthalen-1-yl)acetamide of formula (IV). This compound may be isolated by crystallization after addition of isopropanol and water, in particular in a volume ratio of isopropanol to water of from 2:1 to 1:1, followed by filtration.

The process of step ii) provides *N*-((1*R*,4*S*)-4-(3,4-dichlorophenyl)-3,4-dihydronaphthalen-1-yl)acetamide of formula (IV) in high yield, chemical purity and diastereomeric purity.

The last step of the process, step iii) is deacetylating *N*-((1*R*,4*S*)-4-(3,4-dichlorophenyl)-3,4-dihydronaphthalen-1-yl)acetamide of formula (IV) in the presence of a deacetylating agent, thereby obtaining dasotraline or a pharmaceutically acceptable salt thereof.

This step corresponds to the removal of the acetyl group of acetamide (IV) to give the corresponding amine group of dasotraline (III).

The term "deacetylating agent" refers to a compound, mixture of compounds or entities capable of removing an acetyl group from an acetamide to give the corresponding amine. Examples of deacetylating agents include acids, bases and enzymes. Suitable acids are HCl, or mixtures of methanesulfonic acid and HBr. Other mixtures of compounds or entities capable of removing the acetyl group, i.e. "deacetylating agents", are triphenylphosphite/halogen (e.g. bromine or chlorine) complex and di-*tert-*butyldicarbonate/lithium hydroxide. Other mixtures of compounds or entities capable of removing the acetyl group encompass an activating agent, such as trifluoromethanesuflonic anhydride, phosgene, or oxalyl chloride/pyridine followed by quenching with alcohol, preferably a glycol such as propyleneglycol.

Preferably, the deacetylating agent in step iii) is HCl.

Step iii) may be carried out in any suitable solvent, which can be easily determined by the skilled person. Preferably, the solvent is selected from water, C₁-C₄ alkanol and mixtures thereof. The term "C₁-C₄ alkanol" is as previously defined herein. Examples of C₁-C₄ alkanol are methanol, ethanol, *n*-propanol, isopropanol, *n*-butanol, isobutanol, *sec*-butanol, *tert*-butanol and mixtures thereof, preferably isopropanol, methanol and mixtures thereof, more preferably isopropanol. More preferably the solvent is a mixture of C₁-C₄ alkanol and water, even more preferably isopropanol, methanol and water, even more preferably of isopropanol and water, in particular when the deacetylating agent is HCl (in this case water may be provided as a mixture with HCl).

Preferably, step iii) is carried out at a temperature of from 80 °C to 100 °C, preferably from 85 °C to 92 °C. Preferably, said temperature is kept for 12 h to 48 h, more preferably from 12 h to 36 h, even more preferably from 20 h to 30 h.

When an acid is used as deacetylating agent, the resulting compound is the corresponding dasotraline salt of the acid used. In particular, when HCl is used, dasotraline hydrochloride is obtained, which is a pharmaceutically acceptable salt of dasotraline. The resulting compound can be isolated by filtration.

The process of the invention provides dasotraline or a pharmaceutically acceptable salt thereof in high yield, chemical purity and diastereomeric purity.

The term "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

Further, the term "pharmaceutically acceptable salt" refers to any salt, which, upon administration to the recipient is capable of providing (directly or indirectly) a compound as described herein. Due to the nature of the chemical groups in dasotraline, the pharmaceutically acceptable salt thereof may be acid addition salts, and they can be synthesized from the parent compound which contains a basic moiety by conventional chemical methods. Generally, such salts are, for example, prepared by reacting the free base form of this compound with a stoichiometric amount of the appropriate acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, benzoate, maleate, fumarate, citrate, lactate, glycolate, gluconate, oxalate, pamoate, succinate, tartrate, malate, mandelate, malonate, adipate, acorbate, benzenosulphonate, methanesulphonate, p-toluenesulphonate, naphthalenosulphonate, and the like.

Preferably, the pharmaceutically acceptable salt of dasotraline is the hydrochloric acid salt, i.e. dasotraline hydrochloride.

The acid salt may be converted into the free base by treatment with a base, e.g. by mixing the dasotraline acid salt with water, a suitable base (such as ammonia, sodium hydroxide, potassium hydroxide, sodium carbonate, potassium carbonate, cesium carbonate, and the like) and a non-water-miscible organic solvent, followed by separation of the organic phase and removal of the solvent. The resulting dasotraline (free base) may also be converted into a pharmaceutically acceptable salt thereof using a procedure as explained above.

The following examples represent specific embodiments of the present invention. They do not intend to limit in any way the scope of the invention defined in the present description.

### Examples

### Chromatographic methods

### Method for determining purity by Ultra High Performance Liquid Chromatography (UHPLC)

The purity of compounds obtained in examples 1 and 2 was measured using a HSS T3 C18 2.1 x 100 mm, 1.8 µm column at 40 °C. The chromatograph was equipped with UV detector working at 215 nm. The flow rate was 0.25 mL/min. Test samples were prepared by dissolving the appropriate amount of sample (1 mg/mL) in 50:50 acetonitrile/water and the volume injected of sample was 1 µL.

The mobile phase A was a 0.010 M solution of K₂HPO₄, pH 7.0. The mobile phase B was acetonitrile. The chromatograph was programmed as follow:

| *t* | *%A* | *%B* |
|---|---|---|
| 0.0 | 60 | 40 |
| 0.5 | 60 | 40 |
| 6.0 | 30 | 70 |
| 8.0 | 30 | 70 |
| 10.0 | 60 | 40 |
| 12.0 | 60 | 40 |

The purity of (1*R*,4*S*)-4-(3,4-dichlorophenyl)-1,2,3,4-tetrahydronaphthalen-1-amine hydrochloride obtained in the example 3 was measured using a BEH Shield C18 2.1 x 50 mm, 1.7 µm column at 40 °C. The chromatograph was equipped with UV detector working at 215 nm. The flow rate was 0.25 mL/min. Test samples were prepared by dissolving the appropriate amount of sample (1 mg/mL) in 50:50 acetonitrile/water and the volume injected of sample was 1 µL.

The mobile phase A was a 0.010 M solution of K₂HPO₄, pH 6.3. The mobile phase B was acetonitrile. The chromatograph was programmed as follow:

| *t* | *%A* | *%B* |
|---|---|---|
| 0.0 | 70 | 30 |
| 0.5 | 70 | 30 |
| 6.0 | 30 | 70 |
| 8.0 | 30 | 70 |
| 10.0 | 70 | 30 |
| 12.0 | 70 | 30 |

### Method for determining chiral purity by UHPLC

The chiral purity of (1*R*,4*S*)-4-(3,4-dichlorophenyl)-1,2,3,4-tetrahydronaphthalen-1-amine hydrochloride obtained in the example 3 was measured using a Chiralpack IC 4.6 x 250 mm, 5 µm column at 10 °C. The chromatograph was equipped with UV detector working at 215 nm. The flow rate was 0.7 mL/min. Test samples were prepared by dissolving the appropriate amount of sample (1 mg/mL) in 50:10:40 hexane/isopropanol/methyl-*tert*-butylether and the volume injected of sample was 1 µL. The mobile phase was acetonitrile. Chromatograms were run for 45 minutes.

### Example 1. Synthesis of N-((S)-4-(3,4-dichlorophenyl)-3,4-dihydronaphthalene-1-yl)acetamide

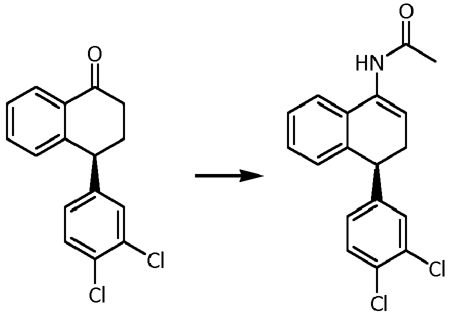

100 g (343.6 mmol) of (*S*)-4-(3,4-dichlorophenyl)-3,4-dihydro-1-naphthalenone were mixed with 74 mL (515.5 mmol) of a 7N solution of NH₃ in methanol to a temperature of about 0°C. Maintaining the indicated temperature, 203 mL (686 mmol) of Ti(OiPr)₄ were slowly added. The resulting mixture was heated to a temperature of about 20 °C and it was stirred for 48 hours at the temperature of between 20 and 25 °C.

The reaction mixture was then cooled to a temperature of about 0 °C and 191 mL (1374 mmol) of triethylamine and 65 mL (686 mmol) of acetic anhydride were consecutively added while keeping the temperature between 0 and 5 °C. The resulting mixture was heated to a temperature of about 20 °C and it was stirred for 24 hours at the temperature of between 20 and 25 °C.

155 mL (721.6 mol) of *N,N,N'N'-*tetrakis(2-hydroxyethyl)ethylenediamine were then slowly added at a temperature of between 20 and 25 °C and the resulting mixture was stirred for 1 hour at said temperature. 900 mL of water, 300 mL of NH₃ and 1000 mL of ethyl acetate were then consecutively added. The resulting mixture was filtered through diatomaceous earth and the organic layer was separated from the aqueous layer. The solvent was vacuum-distilled and 300 mL of toluene and 250 mL of heptane were added to the residue. The resulting mixture was heated to the reflux temperature. The resulting solution was maintained at said temperature for 15 minutes and then slowly cooled to the temperature of about 0 °C. The resulting mixture was maintained at said temperature for 1 hour and the solid was filtered, washed with a portion of 500 mL of a 30:70 mixture of toluene/heptane and finally dried at a temperature of 50 °C to obtain 97.7 g (yield 85.6%) of a crystalline solid corresponding to *N*-((*S*)-4-(3,4-dichlorophenyl)-3,4-dihydronaphthalene-1-yl)acetamide (UHPLC purity: 99.96%).

### Example 2. Synthesis of N-((1R,4S)-4-(3,4-dichlorophenyl)-1,2,3,4-tetrahydronaphthalene-1-yl)acetamide

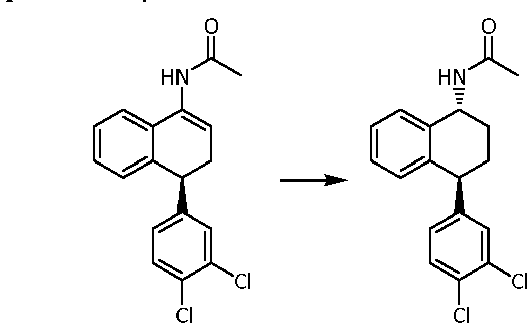

21 g (63.21 mmol) of *N*-((*S*)-4-(3,4-dichlorophenyl)-3,4-dihydronaphthalene-1-yl)acetamide were slurred with 100 mL of isopropanol and the mixture was purged by means of two cycles of vacuum/nitrogen. A previously prepared solution of 110 mg (0.15 mmol) of (*R,R*)-Norphos(COD)Rh-BF₄ (i.e. ((2*R*,3*R*)-(+)-2,3-bis(diphenylphosphino)bicyclo[2.2.1]hept-5-en-(1,5-cyclooctadien)rhodium(I) tetrafluoroborate) in 2.5 mL of methanol was added at a temperature of about 20 °C. The resulting mixture was purged twice with hydrogen and kept stirring at 6 bars of hydrogen and at a temperature of 30±2 °C for 14 hours.

After the hydrogenation was complete, 150 mL of isopropanol were added and the mixture was heated to the reflux temperature and maintained for 30 minutes at said temperature. The obtained solution was cooled at a temperature of about 65 °C and 90 mL of water were slowly added at said temperature. The mixture was then slowly cooled at a temperature of about 0 °C and stirred for 2 hours. The solid was filtered, washed with a portion of 30 mL of isopropanol and finally dried at a temperature of 50 °C to obtain 19.3 g (yield 91.5%) of a crystalline solid corresponding to *N*-((1*R*,4*S*)-4-(3,4-dichlorophenyl)-1,2,3,4-tetrahydronaphthalene-1-yl)acetamide (UHPLC purity: 99.92%).

### Example 3. Synthesis of (1R,4S)-4-(3,4-dichlorophenyl)-1,2,3,4-tetrahydronaphthalen-1-amine hydrochloride (dasotraline hydrochloride)

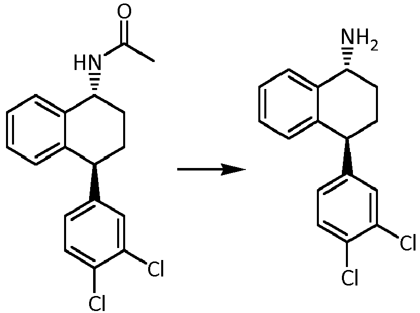

14 g (41.9 mmol) of *N*-((1*R*,4*S*)-4-(3,4-dichlorophenyl)-1,2,3,4-tetrahydronaphthalene-1-yl)acetamide, 70 mL of isopropanol and 40 mL of concentrated hydrochloric acid (37%, 405 mmol) were slurried at a temperature of about 20 °C. The resulting solution was heated to the reflux temperature (about 87 °C) and kept at said temperature for about 24 hours.

After the hydrolysis was complete, the resulting mixture was slowly cooled at a temperature of about 0 °C and maintained at said temperature for 1 hour. The solid was filtered, washed with a portion of 25 mL of isopropanol and finally dried at a temperature of 50 °C to obtain 11.5 g (yield 83.3%) of a white solid corresponding to (1*R*,4*S*)-4-(3,4-dichlorophenyl)-1,2,3,4-tetrahydronaphthalen-1-amine hydrochloride (UHPLC purity: 99.86%; UHPLC chiral purity: the three corresponding diastereoisomers ((1*S*,4*R*), (1*S*,4*S*) and (1*R*,4*R*)) were not detected using the developed method of analysis).

## Claims

1. Process for the preparation of *N*-((*S*)-4-(3,4-dichlorophenyl)-3,4-dihydronaphthalen-1-yl)acetamide of formula (I) 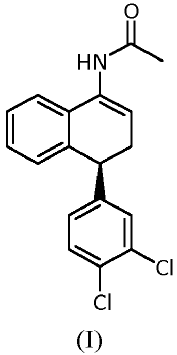 comprising:
a) reacting (*S*)-4-(3,4-dichlorophenyl)-3,4-dihydro-1-naphthalenone of formula (II) with an ammonia source in the presence of a titanium(IV) C₂-C₄ alkoxide 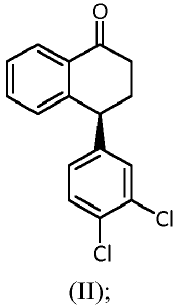
b) reacting the product obtained step a) with an acetyl donor in the presence of a base selected from the group consisting of C₁-C₄ alkylamine and C₁-C₄-dialkylamine to give the *N*-((*S*)-4-(3,4-dichlorophenyl)-3,4-dihydronaphthalen-1-yl)acetamide of formula (I) and
c) optionally isolating the *N*-((*S*)-4-(3,4-dichlorophenyl)-3,4-dihydronaphthalen-1-yl)acetamide of formula (I) obtained in step b).

2. Process according to claim 1, wherein:
- the molar ratio of titanium(IV) C₂-C₄ alkoxide to (*S*)-4-(3,4-dichlorophenyl)-3,4-dihydro-1-naphthalenone of formula (II) used in step a) is from 1.5:1 to 2.5:1;
- the molar ratio of ammonia source to (*S*)-4-(3,4-dichlorophenyl)-3,4-dihydro-1-naphthalenone of formula (II) used in step a) is from 1.1:1 to 2.0:1;
- the molar ratio of acetyl donor used in step b) to (*S*)-4-(3,4-dichlorophenyl)-3,4-dihydro-1-naphthalenone of formula (II) used in step a) is from 1.5:1 to 2.5:1; and/or
- the molar ratio of base used in step b) to (*S*)-4-(3,4-dichlorophenyl)-3,4-dihydro-1-naphthalenone of formula (II) used in step a) is from 2:1 to 5:1.

3. Process according to any of the preceding claims, wherein the titanium(IV) C₂-C₄ alkoxide is titanium(IV) isopropoxide.

4. Process according to any of the preceding claims, wherein the ammonia source is ammonia.

5. Process according to any of the preceding claims, wherein the acetyl donor in step b) is acetic anhydride and the base is triethylamine.

6. Process according to any of the preceding claims, wherein step a) and/or step b) is carried out in a solvent selected from C₁-C₄ alkanol, preferably methanol.

7. Process according to any of the preceding claims, wherein step c) is carried out and comprises:
c1) treating the reaction mixture obtained in step b) with *N,N,N',N'*-tetrakis(2-hydroxy-C₂-C₃ alkyl)ethylenediamine;
c2) adding a basic aqueous solvent and a non-water-miscible organic solvent;
c3) separating the organic phase;
c4) removing the solvent from the organic phase, thereby obtaining isolated *N*-((*S*)-4-(3,4-dichlorophenyl)-3,4-dihydronaphthalen-1-yl)acetamide of formula (I); and
c5) optionally recrystallizing the isolated *N*-((*S*)-4-(3,4-dichlorophenyl)-3,4-dihydronaphthalen-1-yl)acetamide of formula (I).

8. Process according to claim 7, wherein the molar ratio of *N,N,N',N'*-tetrakis(2-hydroxy-C₂-C₃ alkyl)ethylenediamine used in step c1) to titanium(IV) C₂-C₄ alkoxide used in step a) is from 1.0:1 to 2.0:1.

9. Process according to any of claims 7 or 8, wherein the *N,N,N',N'*-tetrakis(2-hydroxy-C₂-C₃ alkyl)ethylenediamine is *N,N,N',N'*-tetrakis(2-hydroxyethyl)ethylenediamine.

10. Process according to any of claims 7 to 9, wherein the basic aqueous solvent of step c2) is a mixture of water and ammonia and the non-water-miscible organic solvent of step c2) is ethyl acetate.

11. Process according to any of claims 7 to 10, wherein the recrystallization of step c5) is carried out in a mixture of heptane and toluene.

12. Process for the preparation of dasotraline of formula (III) 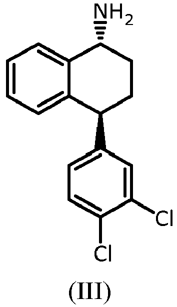 or a pharmaceutically acceptable salt thereof, said process comprising:
i) carrying out the process defined in any of claims 1 to 11, thereby obtaining *N*-((*S*)-4-(3,4-dichlorophenyl)-3,4-dihydronaphthalen-1-yl)acetamide of formula (I) 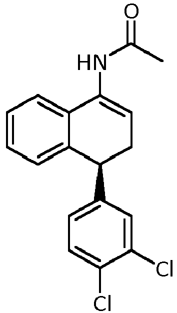 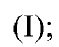
ii) hydrogenating the *N*-((*S*)-4-(3,4-dichlorophenyl)-3,4-dihydronaphthalen-1-yl)acetamide of formula (I) obtained in step i) with hydrogen in the presence of a chiral hydrogenation catalyst, thereby obtaining *N*-((1*R*,4*S*)-4-(3,4-dichlorophenyl)-3,4-dihydronaphthalen-1-yl)acetamide of formula (IV) 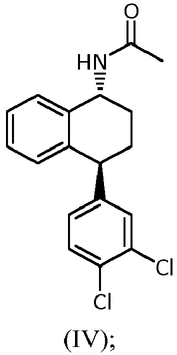 and
iii) deacetylating *N*-((1*R*,4*S*)-4-(3,4-dichlorophenyl)-3,4-dihydronaphthalen-1-yl)acetamide of formula (IV) in the presence of a deacetylating agent, thereby obtaining dasotraline or a pharmaceutically acceptable salt thereof.

13. Process according to claim 12, wherein the molar ratio of chiral hydrogenation catalyst to N-((S)-4-(3,4-dichlorophenyl)-3,4-dihydronaphthalen-1-yl)acetamide of formula (I) used in step ii) is from 0.00125:1 to 0.005:1, preferably from 0.002:1 to 0.005:1.

14. Process according to any of claims 12 or 13, wherein the chiral hydrogenation catalyst of step ii) is ((2*R*,3*R*)-(+)-2,3-bis(diphenylphosphino)bicyclo[2.2.1]hept-5-en-(1,5-cyclooctadien) rhodium(I) tetrafluoroborate and/or step ii) is carried out in the presence of a solvent selected from a C₁-C₄ alkanol, preferably selected from the group consisting of isopropanol, methanol and mixtures thereof.

15. Process according to any of claims 11 to 14, wherein the deacetylating agent in step iii) is HCl, thereby obtaining dasotraline hydrochloride.
